(19) Europäisches Patentamt
European Patent Office
Office européen des brevets

(11) Veröffentlichungsnummer: **0 019 771**
**B1**

# (12) EUROPÄISCHE PATENTSCHRIFT

(45) Veröffentlichungstag der Patentschrift: **16.06.82**

(51) Int. Cl.³: **C 07 D 313/04,** C 09 K 15/06

(21) Anmeldenummer: **80102518.0**

(22) Anmeldetag: **08.05.80**

(54) **Stabilisierung von Caprolactonen.**

(30) Priorität: **23.05.79 DE 2920847**

(43) Veröffentlichungstag der Anmeldung: **10.12.80 Patentblatt 80/25**

(45) Bekanntmachung des Hinweises auf die Patenterteilung: **16.06.82 Patentblatt 82/24**

(84) Benannte Vertragsstaaten: **BE DE FR GB NL SE**

(56) Entgegenhaltungen:
**DE-A-1 669 687**
**DE-A-2 160 405**
**US-A-3 227 730**
**US-A-3 274 216**

(73) Patentinhaber: **Degussa Aktiengesellschaft, Weissfrauenstrasse 9, D-6000 Frankfurt am Main 1 (DE)**
Patentinhaber: **BAYER AG, Zentralbereich Patente, Marken und Lizenzen, D-5090 Leverkusen 1, Bayerwerk (DE)**

(72) Erfinder: **Klenk, Herbert, Dr., Gruenaustrasse 19, D-6450 Hanau 9 (DE)**
Erfinder: **Wirthwein, Rolf, Dr., Fuerstenbergstrasse 4, D-6450 Hanau 9 (DE)**
Erfinder: **Waldmann, Helmut, Dr., Carl-Rumpff-Strasse 59, D-5090 Leverkusen (DE)**
Erfinder: **Seifert, Hermann, Dr., Ruwergasse 4, D-5000 Köln 60 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Stabilisierung von Caprolactonen

Die vorliegende Erfindung bezieh: sich auf das Verhindern einer unerwünschten Farbentwicklung in Caprolactonen, bevorzugt ε-Caprolacton, und erreicht damit eine Stabilisierung dieser wichtigen technischen Verbindungen.

Bekanntlich liegt einer der Hauptverwendungszwecke, vor allem von ε-Caprolacton, in der Herstellung von chemischen Zwischenprodukten, wie z.B. von Polycaprolactonpolyolen. Derartige Polyole stellen das Ausgangsprodukt bei der Herstellung von Polyurethanen dar, die eine breite Verwendung finden. Bei vielen dieser Anwendungsgebiete, wie z.B. Polyurethanfasern und -überzügen, wird vom Verbraucher verlangt, dass die Polyurethanfasern und -überzüge praktisch farblos sind.

Aufgrund der bekannten Eigenschaften der Caprolactone, vor allem ε-Caprolacton, sich beim Lagern nach mehr oder weniger kurzer Zeit zu verfärben, treten hier grosse Schwierigkeiten auf, denn die aus einem solchen Caprolacton hergestellten Polyesterole und Polyurethane sind ebenfalls gefärbt.

Es ist daher technisch von grosser Bedeutung, wirklich farbstabile Caprolactone zu gewinnen.

Um zusätzliche Reinigungsoperationen — vor allem bei frischem oder gealtertem ε-Caprolacton — zu vermeiden, wie z.B. Destillationen oder chemische Behandlungen, ging man dazu über, bestimmte organische Stoffe als Stabilisatoren für Caprolactone einzusetzen.

So ist es z.B. nach der US-Patentschrift 3 227 730 bekannt, Triorganophosphite als Stabilisatoren für monomere Caprolactone zu verwenden. Erwähnt werden in dieser Patentschrift ausserdem tert.-Butyl gehinderte Phenole wie auch Monoalkyläther von Hydrochinon, z.B. sein Monomethyläther, als Stabilisatoren für ε-Caprolacton. Der Stabilisierungseffekt war aber nach Angaben der US-Patentschrift 3 274 216 nicht ausreichend, so dass nach dem Verfahren dieser Patentschrift ein Gemisch aus Triorganophosphiten und Alkylphenolen empfohlen wurde. Die Stabilisierungen wurden vorwiegend in einer Inertgasatmosphäre, die aus sehr reinem Stickstoff bestand, vorgenommen. Luft konnte zwar auch angewendet werden, wurde aber als nicht so günstig angesehen.

Nach Ansicht der DT-OS 2 160 405 beeinflusst eine solche Mischung aus Alkylphenolen und Triorganophosphiten bzw. ein Zusatz von Triorganophosphiten allein die physikalischen Eigenschafte:ı von daraus hergestelltem Polyurethanprodukt in nachteiliger Weise, so dass die Zugabe einer inhibierenden Menge von Triorganophosphin- oder Triorganophosphinitverbindungen vorgeschlagen wurde.

Auch Triphenylphosphin wird als Stabilisator eingesetzt, s. DT-AS 2 215 909, und dabei die Stabilisierung in einer Inertgasatmosphäre wie Methan, Erdgas, einem Edelgas oder Stickstoff vorgenommen.

Von den Stabilisatoren und Stabilisatorgemischen wurden bisher bevorzugt Mengen von 250 - 1000 ppm verwendet.

Die angeführten Verfahren sind also dadurch gekennzeichnet, dass die Stabilisierung von ε-Capro'acton vorwiegend in einer Inertgasatmosphäre vorgenommen wird, da es bekanntlich nicht immer einfach ist, Sauerstoff oder Luft von Gefässen, wie Reaktorteilen, Rührkesseln, Mischbehältern, Lager- und Transportgebinden fernzuhalten. Insbesondere das Abfüllen und Wiederverschliessen auch kleinerer Einheiten erfordert einen erheblichen technischen Aufwand, wenn dies unter vollkommenem Luftausschluss geschehen ist.

Es ist also technisch von grosser Bedeutung und Ziel der Erfindung, die Stabilisierung von Caprolactonen, vor allem ε-Caprolacton, ohne jede Beeinträchtigung direkt in Luft vorzunehmen.

Es wurde nun gefunden, dass diese Art der Stabilisierung erreicht werden kann, wenn man den Caprolactonen, vor allem ε-Caprolacton, Hydrochinon und/oder Dihydroxybenzole der Formel I und/oder II

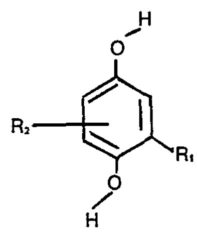 (I) 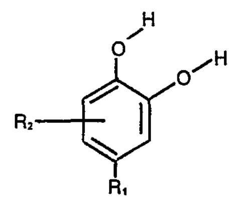 (II)

in denen $R_1$ und $R_2$ eine Alkylgruppe mit 1 - 8 Kohlenstoffatomen bedeuten, wobei $R_1$ und $R_2$ gleich oder verschieden sein können und $R_2$ auch Wasserstoff bedeuten kann, in Mengen von 50 - 500 ppm zusetzt.

Bevorzugt ist eine Alkylgruppe mit 3 - 8 Kohlenstoffatomen, die am α-Kohlenstoffatom verzweigt ist.

Es können auch Gemische solcher Verbindungen verwendet werden. Die Stabilisatoren werden im allgemeinen mit den Caprolactonen vermischt, und zwar bevorzugt in Gegenwart von Luft.

Bevorzugt sind diejenigen Verbindungen, in denen $R_1$ eine tert.-Alkylgruppe, wie z.B. tert.-Butyl und $R_2$ = Wasserstoff ist. So kommen entsprechende Derivate von Brenzkatechinen, z.B. 4-tert.-Butylbrenzcatechin, in Frage.

Das Vermischen des Stabilisators mit dem Caprolacton erfolgt in üblichen Apparaturen, wie z.B. Mischkesseln.

Das Verfahren wird bevorzugt mit frisch destilliertem ε-Caprolacton durchgeführt, vorzugsweise kommen Mengen zwischen 100 - 500 ppm in Betracht.

Das erfindungsgemässe Verfahren wird an folgenden Beispielen erläutert:

Beispiele

In allen Fällen wurden 100 g frisch destilliertes ε-Caprolacton in ein 250 ml Becherglas gegeben und die berechnete Menge Stabilisator hinzugefügt. Dann wurde das offene Becherglas in einen Wärmeschrank gestellt, wo es bei einer Temperatur von 100°C für 24 Stunden verblieb und anschliessend die Farbzahl des ε-Caprolactons nach der ASTM-Methode D 1209-54 (Pt-Co) bestimmt.

Tabelle 1 gibt die erhaltenen Ergebnisse wieder. Dabei stellen die Beispiele 1 - 5 Vergleichsbeispiele mit Stabilisatoren nach dem Stand der Technik dar.

Beispiele 6 und 7 erläutern die Erfindung.

Tabelle 1

| Probe Nr. | eingesetzter Stabilisator | Menge | Farbzahl des Caprolactons |
|---|---|---|---|
| 1 | ohne Stabilisator | — | ≫500 |
| 2 | 2,6 Di-tert.-butylphenol | 100 ppm | ~500 |
| 3 | Phosphorigsäure-tributylester | 100 ppm | >500 |
| 4 | Mischung aus a) 2,6 Di-tert.-butylphenol b) Phosphorigsäure-tributylester | 50 ppm<br>50 ppm | 400 |
| 5 | Triphenyl-phosphin | 100 ppm | 500 |
| 6 | tert.-Butyl-hydrochinon | 100 ppm | 10 - 20 |
| 7 | 4-tert.-Butyl-brenzcatechin | 100 ppm | 10 - 20 |

Die genannten Stabilisatoren, die sich durch ihre Einsatzmöglichkeit in Gegenwart von Luft auszeichnen, können selbstverständlich auch in üblicher Inertgasatmosphäre benutzt werden.

**Patentansprüche**

1. Verfahren zum Stabilisieren von Caprolactonen, dadurch gekennzeichnet, dass man den Lactonen Hydrochinon und/oder Dihydroxybenzole der Formel I und/oder II

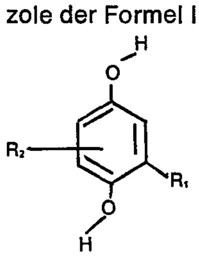

(I)

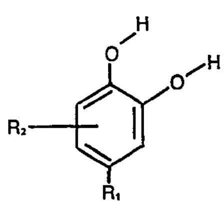

(II)

in denen $R_1$ und $R_2$ eine Alkylgruppe mit 1 - 8 Kohlenstoffatomen bedeuten, wobei $R_1$ und $R_2$ gleich oder verschieden sein können und $R_2$ auch Wasserstoff bedeuten kann, in Mengen von 50 - 500 ppm zusetzt.

2. Verfahren nach Anspruch 1, dadurch gekennzeichnet, dass $R_1$ und $R_2$ eine Alkylgruppe mit 3 - 8 Kohlenstoffatomen bedeuten, die am α-Kohlenstoffatom verzweigt ist.

3. Verfahren nach Anspruch 1 und 2, dadurch gekennzeichnet, dass man den Stabilisator in einer Menge von 100 bis 500 ppm, bezogen auf das Caprolacton, einsetzt.

4. Verfahren nach Anspruch 1 bis 3, dadurch gekennzeichnet, dass man den Lactonen den Stabilisator zusetzt, indem man das Lacton mit dem Stabilisator in Gegenwart von Luft vermischt.

5. Verfahren nach Anspruch 1 bis 4, dadurch gekennzeichnet, dass man den Stabilisator dem ε-Caprolacton zusetzt.

**Claims**

1. A process for stabilizing caprolactones, characterised in that hydroquinone and/or dihydroxybenzenes of the following formula I and/or II:

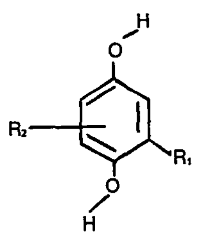

(I)

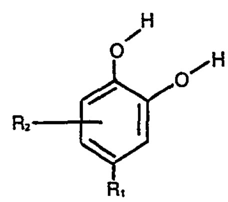

(II)

wherein

$R_1$ and $R_2$ may be the same or different and represent an alkyl group having from 1 to 8 carbon atoms, and $R_2$ may also represent hydrogen, are added to the lactones in quantities of from 50 to 500 ppm.

2. A process according to claim 1, characterised in that $R_1$ and $R_2$ represent an alkyl group having from 3 to 8 carbon atoms which is branched at the α-carbon atom.

3. A process according to claims 1 and 2, characterised in that the stabilizer is used in a quantity of from 100 to 500 ppm, based on the caprolactone.

4. A process according to claims 1 to 3, characterised in that the stabilizer is added to the lactones by mixing the lactone with the stabilizer in the presence of air.

5. A process according to claims 1 to 4, characterised in that the stabilizer is added to the ε-caprolactone.

**Revendications**

1. Procédé pour la stabilisation de caprolactones, caractérisé en ce que l'on ajoute aux

lactones de l'hydroquinone et/ou du dihydroxybenzène de formules I et/ou II

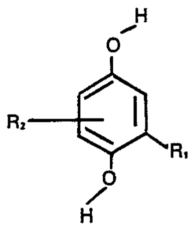

(I)

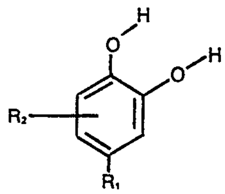

(II)

dans lesquelles $R_1$ et $R_2$ signifient un groupe alcoyle avec 1 à 8 atomes de carbone, $R_1$ et $R_2$ pouvant être identiques ou différents, et $R_2$ pouvant également représenter de l'hydrogène, en quantités de 50 - 500 ppm.

2. Procédé selon la revendication 1, caractérisé en ce que $R_1$ et $R_2$ représentent un groupe alcoyle avec 3 - 8 atomes de carbone, qui est ramifié au niveau de l'atome de carbone.

3. Procédé selon l'une des revendications 1 et 2, caractérisé en ce que l'on introduit le stabilisant en quantité de 100 à 500 ppm, rapporté au caprolactone.

4. Procédé selon l'une quelconque des revendications 1 à 3, caractérisé en ce que l'on ajoute le stabilisant au lactone, en mélangeant le lactone avec le stabilisant en présence d'air.

5. Procédé selon l'une quelconque des revendications 1 à 4, caractérisé en ce que l'on ajoute le stabilisant au ε-caprolactone.